## Europäisches Patentamt
## European Patent Office
## Office européen des brevets

(19)

(11) Veröffentlichungsnummer: **0 283 823**
**B1**

(12) **EUROPÄISCHE PATENTSCHRIFT**

(45) Veröffentlichungstag der Patentschrift:
03.10.90

(51) Int. Cl.⁵: **B06B 1/02,** G10K 11/00, A61H 23/00

(21) Anmeldenummer: **88103536.4**

(22) Anmeldetag: **07.03.88**

(54) **Gerät zur Erzeugung und Abstrahlung von Ultraschall,insbesondre für Ultraschall-Therapie.**

(30) Priorität: **20.03.87 DE 3709110**

(43) Veröffentlichungstag der Anmeldung:
**28.09.88 Patentblatt 88/39**

(45) Bekanntmachung des Hinweises auf die Patenterteilung:
**03.10.90 Patentblatt 90/40**

(84) Benannte Vertragsstaaten:
**AT BE CH DE GB LI NL**

(56) Entgegenhaltungen:
EP-A- 0 176 136
DE-A- 3 324 575
US-A- 3 727 112
US-A- 3 736 523
US-A- 4 181 864
US-A- 4 525 790

(73) Patentinhaber: **Siemens Aktiengesellschaft, Wittelsbacherplatz 2, D-8000 München 2(DE)**

(72) Erfinder: **Herzog, Ludwig, Drei-Thorn-Strasse 1, D-6948 Waldmichelbach(DE)**
Erfinder: **Knapp, Volker, Pestalozzistrasse 19, D-6948 Waldmichelbach(DE)**

## Beschreibung

Die Erfindung bezieht sich auf ein Gerät zur Erzeugung und Abstrahlung von Ultraschall gemäß Oberbegriff des Patentanspruches 1. Der Ultraschallkopf kann dabei auch z.B. für kombinierte Ultraschall- und Reizstromtherapie ausgelegt sein.

Ein Gerät dieser Art ist zum Beispiel durch die EP-A 0 176 136 vorbekannt. Der Schallkopf dieses Gerätes umfaßt zwei Widerstandspotentiometer, die durch Verstellen des veränderbaren Kontaktes eine Anpassung zwischen dem jeweils eingesetzten Ultraschallkopf und dem elektrischen Schwingungserzeuger erlauben. Wenn die Einstellung stattgefunden hat, kann jeder dem Gerät angepaßte Schallkopf verwendet werden und können defekte Schallköpfe ohne weiteres durch ebenfalls angepasste ersetzt werden. Ein ähnliches Gerät ist auch schon durch den Prospekt "Impulsaphon-U, Dr. Born GmbH, Ultraschall-Therapiegerät M100", aus dem Jahre 1983 vorbekannt. Ein Ultraschallkopf mit einem Ultraschallschwinger für Ultraschall-Therapie, der allerdings kein Anpaßglied im Kopf umfaßt, ist auch noch durch die DE-A 33 24 575 vorbekannt.

Bei den bekannten, mittels Widerstandspotentiometer anpaßbaren Geräten wird die Anpassung gewöhnlich im Werk vorgenommen. Eine Anpassung direkt am Einsatzort, z.B. Klinik oder ärztliche Praxis, ist wegen des benötigten technischen Aufwandes schwierig.

Aufgabe vorliegender Erfindung ist es, ein Gerät der eingangs genannten Art dahingehend auszubilden, daß eine Anpassung direkt am Einsatzort (z.B. durch Servicetechniker) rasch und ohne großen technischen Aufwand möglich ist.

Die Aufgabe wird erfindungsgemäß durch die kennzeichnenden Merkmale des Patentanspruchs 1 gelöst.

Gemäß der Erfindung bilden jetzt Anpassungsorgan und auswechselbar einsetzbarer Ultraschallschwinger ein immer angepaßtes Paar. Durch einfachen Austausch eines neuen Paares gegen ein altes Paar wird der Ultraschallkopf am Einsatzort automatisch an den elektrischen Schwingungserzeuger angepaßt. Abweichungen von der ursprünglichen Anpassung lassen sich also sofort an Ort und Stelle bereinigen.

Weitere Vorteile und Einzelheiten der Erfindung ergeben sich aus der nachfolgenden Beschreibung eines Ausführungsbeispiels anhand der Zeichnung und in Verbindung mit den Unteransprüchen. Es zeigen:

Fig. 1 ein Gerät zur Erzeugung und Abstrahlung von Ultraschall im Prinzipschaltbild, das die Erfindung beinhaltet,

Fig. 2 einen die Erfindung gemäß Fig. 1 enthaltenden Schallkopf, teilweise im Schnitt.

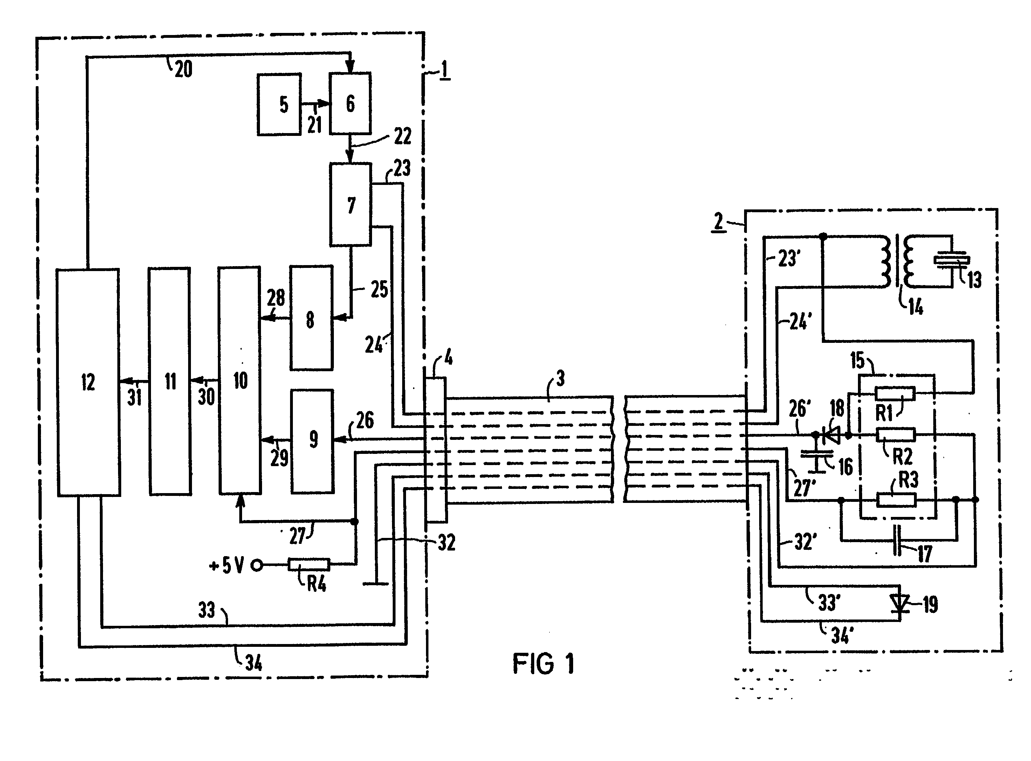

Im Prinzipschaltbild der Fig. 1 ist der elektrische Signalerzeuger mit 1 und der Ultraschallkopf mit 2 bezeichnet. Der Ultraschallkopf 2 ist am elektrischen Signalerzeuger 1 mittels Kabel 3 und Kabelstecker 4 angekoppelt.

Der elektrische Signalerzeuger 1 umfaßt unter anderem einen Oszillator 5 (der z.B. 875 kHz liefert), eine Filter- und Begrenzerschaltung 6, einen Verstärker 7, eine Strommeßeinrichtung 8, eine Spannungserfassungseinrichtung 9, einen Multiplexer 10, einen Analog-Digital-Wandler 11 sowie einen zentralen Prozessor 12.

Der Ultraschallkopf 2 umfaßt einen Ultraschallschwinger 13 mit vorgeschaltetem Breitbandübertrager 14. Er beinhaltet ferner ein aus ohmschen Festwiderständen R1, R2, R3 gebildetes Anpassungsorgan 15, das Bestandteil einer Anpassungsvorrichtung ist, zu der im elektrischen Signalerzeuger auch noch wenigstens teilweise die Bauteile 8 bis 12 gehören.

Die Bauelemente 16, 17 bzw. 18 sind Glättungskondensatoren bzw. eine Diode zur Gleichrichtung. Mit R4 ist wieder ein ohmscher Festwiderstand bezeichnet.

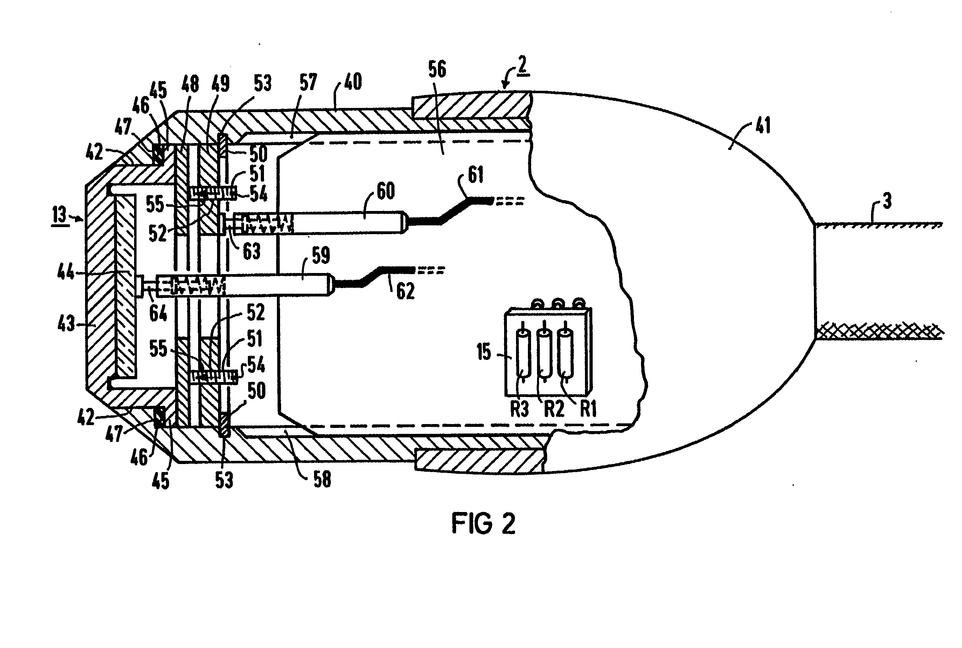

Das Anpassungsorgan 15 besteht im vorliegenden Fall aus einem steckbaren IC-Sockel, auf dem die ohmschen Festwiderstände R1, R2, R3 aufgelötet sind (siehe dazu auch Fig. 2). Das Anpassungsorgan 15 ist also im Sinne der Erfindung leicht auswechselbar.

Der Ultraschallkopf 2 umfaßt auch noch eine Leuchtdiode 19 als Anzeigeelement, daß der Ultraschallkopf über das Kabel 3 und den Kabelstecker 4 richtig am elektrischen Signalerzeuger 1 angekoppelt ist (d.h., daß der Ultraschallschwinger entsprechend der eingestellten Leistung angesteuert wird).

Die jeweilige Schaltungsanordnung im elektrischen Signalerzeuger 1 sowie im Ultraschallkopf 2 beinhaltet lediglich die Schaltungsteile, welche für das Verständnis von Aufbau und Funktionsweise der vorliegenden Erfindung notwendig sind. Dasselbe gilt für die Signalleitungen 20 bis 34 im elektrischen Signalerzeuger 1 bzw. für die korrespondierenden (über Kabelstecker 4 und Kabel 3 angekoppelten) Signalleitungen 23′, 24′, 26′, 27′, 32′ bis 34′ im Ultraschallkopf 2.

Das Gerät nach der Fig. 1 funktioniert in der Weise, daß der Oszillator 5 hochfrequente elektrische Signale erzeugt, die über die Filter- und Begrenzerschaltung 6 und den Verstärker 7 sowie die korrespondierenden Signalleitungen 23, 23′ bzw. 24, 24′ und den Breitbandübertrager 14 dem Ultraschallschwinger 13 des Ultraschallkopfes 2 zugeführt werden. Der Ultraschall schwinger 13 wandelt die zugeführten elektrischen Signale in Ultraschall um und strahlt diesen ab (z.B. in menschliches Körpergewebe bei der Ultraschall-Therapie).

Der Ultraschallkopf 2 und der elektrische Signalerzeuger 1 sind, so lange keine Störungen des Ultraschallschwingers vorliegen (z.B. mechanischer Bruch oder Depolarisierung durch Überhitzung oder elektrolytische Abtragung der Behandlungsoberfläche des Schwingerträgers bei Ultraschall- und Reizstromtherapie) über die Anpassungsvorrichtung 8 bis 12, 15 gut aneinander angepaßt. Die Teilerspannung der Widerstände R1 und R2 wird nämlich in an sich bekannter Weise über die Gleichrichtdiode 18 und den Glättungskondensator 16 der Spannungserfassungseinrichtung 9 zugeleitet. Entsprechend wird über die Parallelschaltung des Widerstandes R3 und des Kondensators 17 ein Korrektursignal gewonnen, das über die Signalleitung 27', 27 (wie auch das Ausgangssignal der Spannungserfassungseinrichtung 9 über die Signalleitung 29) dem Multiplexer 10 zugeleitet werden. Der Multiplexer 10 tastet die zugeführten Signale (ebenso das Ausgangssignal der Strommesseinrichtung 8) abwechselnd ab und gibt das jeweilige Ergebnis über den Analog-Digital-Wandler 11 in den zentralen Prozessor 12 ein. Dieser erzeugt dann durch Vergleich mit eingespeicherten Korrekturdaten ein Signal, das über die Signalleitung 20 die Übertragungseigenschaften der Filter- und Begrenzerschaltung 6 bzw. des Verstärkers 7 im Sinne der erwünschten Anpassung des Ultraschallkopfes 2 an den elektrischen Signalerzeuger 1 verändert.

Tritt nun jedoch eine Störung auf, die ein Auswechseln des Ultraschallschwingers 13 erforderlich macht, so kann die mit dem Auswechseln erforderliche neue Anpassung direkt an Ort und Stelle, z.B. in der Klinik oder in der ärztlichen Praxis, erfolgen. Der Servicetechniker entfernt dazu den alten Ultraschallschwinger sowie das bisherige Anpassungsorgan 15. Als Ersatz wird dann lediglich nur noch ein neues Paar (d.h. neuer Ultraschallschwinger 13 mit dazu passendem neuen Anpassungsorgan 15) eingesetzt. Die zueinander passenden Teile eines jeden Paares werden dazu bereits vom Hersteller bzw. Vertreiber des Gerätes paarweise verpackt, so daß es beim Serviceeinsatz nicht zu Verwechslungen kommen kann. Je nach der Zahl der zu reparierenden Geräte, benötigt also der Servicetechniker am Einsatzort lediglich eine entsprechende Zahl bereits abgepackter Paare von zueinander passenden Ultraschallschwingern 13 und Anpassorganen 15. Weitere Utensilien, wie z.B. aufwendige Meßgeräte oder dgl., sind am Einsatzort nicht erforderlich.

Wie zuvor schon erwähnt, ist das Anpassungsorgan 15 als steckbarer IC-Sockel leicht auswechselbar. Aber auch der Ultraschallschwinger 13 ist leicht auswechselbar, wie anhand der Fig. 2 noch näher erläutert werden soll.

Die Fig. 2 zeigt ein Ausführungsbeispiel für den Ultraschallkopf 2, der die Erfindung umfaßt. Der Ultraschallkopf 2 umfaßt ein proximales Gehäuseteil 40 (z.B. aus Kunststoff) und ein wenigstens teilweise darüberschiebbares distales Gehäuseteil 41 (z.B. ebenfalls aus Kunststoff). In der Fig. 2 ist das proximale Gehäuseteil 40 im wesentlichen im Längsschnitt und das distale Gehäuseteil 41 teilweise aufgebrochen dargestellt.

Das proximale Gehäuseteil 40 besitzt eine applikationsseitige Öffnung 42, in der von innen her der aus Schwingerträger 43 (z.B. Aluminium) und daraufgeklebter Keramikschwingscheibe 44 bestehende Ultraschallschwinger 13 eingesetzt ist. Der Schwingerträger 43 sitzt dabei mit seinem nach außen gekröpften Rand 45 über einen Dichtring 46 auf einer Innenschulter 47 des proximalen Gehäuseteils 40. Zum Anpressen des Schwingerträgers 43 dient eine Anpreßmechanik bestehend aus einer dünnen gelochten Metallscheibe 48 (z.B. Stahl), einer dicken gelochten Metallscheibe 49 (z.B. Stahl), einem Sicherungsring 50 und z.B. insgesamt drei (metallische) Festklemmschrauben 51, die jeweils in einem zugehörigen Gewindeloch 52 der dicken gelochten Metall scheibe 49 senkrecht zu dieser verstellbar angeordnet sind.

Beim Montieren wird im Inneren des proximalen Gehäuseteiles 40 zuerst der Dichtring 46 auf die Innenschulter 47 des proximalen Gehäuseteiles 40 aufgelegt. Anschließend wird der Ultraschallschwinger 13 (Schwingerträger 43 samt Keramikschwingscheibe 44) in der bereits beschriebenen Weise in die applikationsseitige Öffnung 42 eingesetzt. Dann werden die dünne gelochte Metallscheibe 48 und die dicke gelochte Metallscheibe 49 eingelegt und der Sicherungsring 50 in die Ringnut 53 entlang der Innenwand des proximalen Gehäuseteiles 40 eingesetzt. Jede Festklemmschraube 51 besitzt einen Schlitz 54 für einen Schraubenzieher. Durch Drehen mit dem Schraubenzieher bewegt sich nun die jeweilige Festklemmschraube 51 in Richtung des Pfeils 55. Mit dem Sicherungsring 50 als Widerlager für die dicke gelochte Metallscheibe 49 drückt jetzt also die Gesamtzahl aller Festklemmschrauben 51 den Ultraschallschwinger 13 über die dünne gelochte Metallscheibe 48 dichtend in die applikationsseitige Öffnung 42 des proximalen Gehäuseteiles 40.

Das distale Gehäuseteil 41 mit dem angedeuteten Ende des Kabels 3 umfaßt eine gedruckte Schaltungsplatte 56 auf der sich die wesentlichen elektrischen Bauelemente der elektrischen Schaltung des Ultraschallkopfes 2 befinden. Unter anderem ist auf dieser gedruckten Schaltungsplatte 56 auch der IC-Sockel mit den ohmschen Widerständen R1 bis R3 des Anpassungsorgans 15 ansteckbar. Die gedruckte Schaltungsplatte 56 läuft beim Überschieben des distalen Gehäuseteils 51 über das proximale Gehäuseteil 40 entlang zwei inneren Längsnuten 57, 58 des proximalen Gehäuseteiles 40.

Die Bauelemente 59, 60 sind Federkontaktstifte zur Kontaktierung des Ultraschallschwingers 13. Sie sind einerseits auf der Schaltungsplatte 56 angelötet und stehen dort also mit elektrischen Leiterbahnen 61, 62 zur Spannungsversorgung in elektrischem Kontakt. Andererseits stehen sie über federvorge spannte Kontaktfüßchen 63, 64 zum einen mit der Oberfläche der Keramikschwingscheibe 44 und zum anderen über die dicke gelochte Metallscheibe 49, die Festklemmschrauben 51 und die dünne gelochte Metallscheibe 48 mit dem Schwingerträger 43 in Kontakt.

Im Störungsfall wird der defekte Ultraschallschwinger 13 (Schwingerträger 43 samt Keramikschwingscheibe 44) in umgekehrter Weise, wie oben für die Montage beschrieben, demontiert und kann somit leicht durch einen neuen intakten Ultraschallschwinger 13 ersetzt werden.

## Patentansprüche

1. Gerät zur Erzeugung und Abstrahlung von Ultraschall, insbesondere für Ultraschall-Therapie, mit einem Ultraschallkopf, der einen Ultraschallschwinger umfaßt, und mit einem elektrischen Signalerzeuger zur Beaufschlagung des Ultraschallschwingers des Ultraschallkopfes mit elektrischen Signalen in dem Sinne, daß die elektrischen Signale in abzustrahlenden Ultraschall umgewandelt werden, und mit einer Anpassungsvorrichtung zum Anpassen eines eingesetzten Ultraschallkopfes an den elektrischen Signalerzeuger, **dadurch gekennzeichnet,** daß die Anpassungsvorrichtung (8 bis 12, 15) ein leicht auswechselbares, von einem steckbaren IC-Sockel gebildetes Anpassungsorgan (15) umfaßt, das einem ebenfalls leicht auswechselbaren Ultraschallschwinger (13) passend zugeordnet und in den Ultraschallkopf (2) auswechselbar einsetzbar ist, wenn der Ultraschallschwinger ausgewechselt wird.

2. Gerät nach Anspruch 1, **dadurch gekennzeichnet,** daß der Ultraschallschwinger (13) und das dazugehörende Anpassungsorgan (15) durch einen anderen Ultraschallschwinger mit dazugehörigem angepaßtem Anpassungsorgan paarweise auswechselbar ist.

3. Gerät nach Anspruch 1, **dadurch gekennzeichnet,** daß auf dem steckbaren IC-Sockel des Anpassungsorganes (15) Anpaßelemente (R1, R2, R3) angeordnet sind, die auf dem IC-Sockel kontaktierte ohmsche Festwiderstände bilden.

4. Gerät nach Anspruch 1, **dadurch gekennzeichnet,** daß der IC-Sockel auf einer Schaltungsplatte (56) im Ultraschallkopf (2) auswechselbar angesteckt ist.

5. Gerät nach Anspruch 1 und 3, **dadurch gekennzeichnet,** daß die Anpassungsvorrichtung aus dem die ohmschen Festwiderstände (R1, R2, R3) aufweisenden Anpassungsorgan (15) sowie einer Strommeßeinrichtung (8), einer Spannungserfassungseinrichtung (9), einem Multiplexer (10), einem Analog-Digital-Wandler (11) und einem zentralen Prozessor (12) des elektrischen Signalerzeugers (1) besteht.

**Revendications**

1. Appareil pour produire et pour émettre des ultrasons, en particulier pour la thérapie aux ultrasons, avec une tête ultrasonore comprenant un oscillateur ultrasonore, avec un générateur de signaux électriques pour appliquer à l'oscillation ultrasonore des signaux électriques en vue de transformer les signaux électriques en ultrasons à émettre, et avec un dispositif d'adaptation pour l'adaptation au générateur de signaux électriques d'une tête ultrasonore mise en place, caractérisé par le fait que le dispositif d'adaptation (8 à 12, 15) comporte un organe d'adaptation (15) facilement remplaçable, formé par un socle enfichable réalisé en circuits intégrés, et auquel est associé de façon adaptée un oscillateur ultrasonore (13) également remplaçable facilement et susceptible d'être remplacé dans la tête ultrasonore (2) lorsque l'oscillateur ultrasonore est remplacé.

2. Appareil selon la revendication 1, caractérisé par le fait que l'oscillateur ultrasonore (13) avec l'organe d'adaptation (15) qui lui est associé, est susceptible d'être remplacé par paire par un autre oscillateur ultrasonore avec l'organe d'adaptation associé.

3. Appareil selon la revendication 1, caractérisé par le fait que sur le socle enfichable en circuits imprimés de l'organe d'adaptation (15), sont prévus des éléments d'adaptation (R1, R2, R3) qui constituent des résistances ohmiques fixes contactées sur le socle en circuits imprimés.

4. Appareil selon la revendication 1, caractérisé par le fait que le socle en circuits imprimés est monté par enfichage sur une plaquette à circuits imprimés (56) prévue dans la tête ultrasonore (2).

5. Appareil selon les revendications 1 à 3, caractérisé par le fait que le dispositif d'adaptation comporte par un organe d'adaptation (15) qui présente des résistances ohmiques fixes (R1, R2, R3) ainsi qu'un dispositif de mesure du courant (8), un détecteur de tension (9) un multiplexeur (10) un convertisseur analogique-Digital (11) et un processeur central (12) du générateur de signaux électrique (1).

**Claims**

1. Apparatus for generating and radiating ultrasound, in particular for ultrasound therapy, with an ultrasound head, which comprises an ultrasound resonator, and with an electrical signal generator for charging the ultrasound resonator of the ultrasound head with electrical signals in the sense that the electrical signals are converted into ultrasound to be radiated, and with an adaptation device for adapting an employed ultrasound head to the electrical signal generator, characterized in that the adaptation device (8 to 12, 15) comprises an adaptation part (15) which can be exchanged easily, which is formed by a plug-in IC-socket and which is suitably associated with an ultrasound resonator (13), likewise capable of being exchanged easily, and which can be inserted into the ultrasound head (2) so that it can be exchanged, when the ultrasound resonator is exchanged.

2. Apparatus according to claim 1, characterized in that the ultrasound resonator (13) and the associated adaptation part (15) can be exchanged as a pair by a different ultrasound resonator with associated adapted adaptation part.

3. Apparatus according to claim 1, characterized in that adapting elements (R1, R2, R3) are arranged on the plug-in IC-socket of the adaptation part (15) which form ohmic fixed resistances connected to the IC-socket.

4. Apparatus according to claim 1 characterized in that the IC-socket is exchangeably mounted onto a circuit board (56) in the ultrasound head (2).

5. Apparatus according to claims 1 and 3, characterized in that the adaptation device consists of the adaptation part (15), having the ohmic fixed resistances (R1, R2, R3), as well as a current measuring device (8), a voltage detection device (9), a multiplexer (10), an analog-digital converter (11) and a central processor (12) of the electrical signal generator (1).

FIG 1

EP 0 283 823 B1

FIG 2